(19) **Europäisches Patentamt European Patent Office Office européen des brevets**

(11) **EP 4 431 081 A1**

(12) **EUROPEAN PATENT APPLICATION**

(43) Date of publication:
18.09.2024 Bulletin 2024/38

(21) Application number: 23425009.0

(22) Date of filing: 13.03.2023

(51) International Patent Classification (IPC):
$A61K\ 8/35^{(2006.01)}$    $A61K\ 8/365^{(2006.01)}$
$A61K\ 8/368^{(2006.01)}$    $A61K\ 8/37^{(2006.01)}$
$A61K\ 8/40^{(2006.01)}$    $A61K\ 8/46^{(2006.01)}$
$A61K\ 8/49^{(2006.01)}$    $A61Q\ 17/04^{(2006.01)}$

(52) Cooperative Patent Classification (CPC):
A61K 8/368; A61K 8/35; A61K 8/365; A61K 8/37;
A61K 8/40; A61K 8/466; A61K 8/4966; A61Q 17/04

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Prodotti Gianni S.r.l.**
**20138 Milano (IT)**

(72) Inventors:
• **ANSELMI, Cecilia**
 **53100 Siena (IT)**

• **CENTINI, Marisanna**
 **53027 San Quirico d'Orcia (SI) (IT)**
• **COSTA, Jessica**
 **53100 Siena (IT)**
• **POGNI, Rebecca**
 **53100 Siena (IT)**
• **SEGA, Alessandro**
 **50012 Bagno a Ripoli (FI) (IT)**
• **SIGNORI, Giulia**
 **53036 Poggibonsi (SI) (IT)**

(74) Representative: **Botti & Ferrari S.p.A.**
**Via Cappellini, 11**
**20124 Milano (IT)**

(54) **COSMETIC COMPOSITIONS WITH ENHANCED SUN PROTECTION FACTOR AND PREPARATION THEREOF**

(57)    Cosmetic compositions are described comprising combined sunscreens and a water-soluble melanin obtained by enzymatic oxidation of a melanin precursor, preferably selected from gentisic acid, homogentisic acid or mixtures thereof. The combined sunscreens are preferably organic and more preferred combinations thereof are described in the specification. The invention is also directed to a method of preparation of said cosmetic compositions and to the use of said water-soluble melanin as a SPF booster for a cosmetic composition comprising two or more sunscreen.

**Description**

FIELD OF THE INVENTION

The invention belongs to the field of molecules and products useful for sun protection, particularly in the cosmetic field. New combinations of specific types of melanin with various sunscreens are disclosed, whereby the SPF effect of melanin is strongly enhanced.

STATE OF THE ART

Photoprotection is since long in the focus of attention of dermatologists and cosmetologists. Solar radiation is indeed involved in the ageing process and it may represents a co-factor in the development of skin diseases. Cosmetic products include physical and/or chemical sunscreens capable to reflect and/or absorb UV radiation.

Photoprotection is evaluated by determination of SPF (*Sun Protection Factor*), definable as the ratio between the amount of UV energy necessary to produce a minimum erythema (*MED, Minimum Erythemal Dose*) on a protected skin and the corresponding MED on unprotected skin. The official European method for determining SPF in vivo is reported in EN ISO 24444 (2022). At present, the safety of sunscreens has been questioned, in relation to humans and the environment in general.

Since 1st January 2020 the ban on the sale and use of solar products in the Republic of Palau prohibits the use of substances such as Benzophenone-3, Ethylhexyl Methoxycinnamate, 4-Methylbenzylidene Camphor, considered dangerous for the marine ecosystem (corals). Benzophenone-3 and Ethylhexyl Methoxycinnamate were banned in Hawaii in 2021. Clearly it is very important to evaluate the environmental impact of sunscreens in depth, but other paths can also be taken.

SPF boosters are becoming increasingly important; they are molecules capable of increasing the sun protection factor in synergy with sunscreens, allowing to reduce their concentration in the cosmetic. SPF boosters can act via different mechanisms. Most of them increase UV absorption and the thickness of the protective film on the skin, others increase the scattering property of sunscreens, others enhance UVA absorption or improve photostability.

Several SPF boosters from natural or synthetic sources are known and are disclosed in patent publications, e.g. US2008/0219938A1, EP2512434B1, US2011/0256076A1, AU2009307763B2, WO2012/161084A1, WO2015/014818A2, WO2016/07136A1, WO2019/ 024022A1, WO2020/024023A1, WO2020/041391A1, KR 10-1995997, JP5837868B2.

Melanin is a dark brown heteropolymer, high molecular pigment found in animals, plants, bacteria, fungi. Depending on the source and the precursor, there are different types of melanins: eumelanin, pheomelanin, neuromelanin, allomelanin and pyomelanin. In humans, melanins are found in the skin, hair, certain parts of the brain and the retina. There are many literature publications concerning skin pigmentation, wherein endogenous melanin (eumelanin; pheomelanin) is reported to act as an endogenous skin photoprotector.

Melanin can be included in sunscreen products and various publications propose melanins of microbial or extractive origin for their photoprotective action.

Two publications of 2017 (http://dx.doi.org/10.13005/bbra/2594) and 2018 (https://doi.org/10.1016/j.dib.2018.04.123) characterize black melanic pigments produced by marine bacteria *Providencia rettgeri* BTKKS1 and *Pseudomonas stutzeri* BTCZ10 and describe their protective capacity when inserted into commercial sunscreens. The results obtained indicate an increase in SPF ranging from 10% to 21% (2-3 points of SPF) in the case of pyomelanin obtained by *Providencia rettgeri* BTKKS1 and from 7% to 17.5% (2-3 SPF points) in the case of pyomelanin obtained by *Pseudomonas stutzeri* BTCZ10.

Properties of possible cosmetic interest have recently been studied, for example, in pyomelanin from yeast (*Yarrowia lipolytica* W29): this substance was included in commercial sunscreen products such as Nivea Sun and Garnier Ambre Solaire, and its booster effect at various concentrations was reported, i.e. an increase in the sun protection factor from 0% to 39%, depending on the concentration of melanin in the formulation (https://doi.org/10.1002/btpr.2912 ).

A review was published in 2021 regarding microbial melanins and their applications, among which their inclusion in sunscreen products (https://doi.org/10.1016/j.biotechadv.2021.107773).

Two publications of 2018 (https://doi.org/10.1002/adfm.201802127) and 2021 (https://doi.org/10.1016/j.msec.2021.112346) disclose a hydrogel which traps polydopamine nanoparticles and induces resistance to penetration in the skin, superior UV shielding properties and higher photoprotection.

SUMMARY

It has now been observed that the SPF boosting effect of water-soluble melanin in cosmetic compositions can be significantly enhanced if the water-soluble melanin is one obtained via a specific natural method and the cosmetic composition contains a combination of two or more sunscreens. Accordingly, an object of the invention is represented by a cosmetic composition comprising combined sunscreens and a water-soluble melanin obtained by enzymatic oxidation of a melanin precursor. The invention is also directed to the method of preparation of said cosmetic compositions and to the use of said water-soluble melanin as a SPF booster for a cosmetic composition comprising two or more sunscreens.

DESCRIPTION OF THE FIGURES

- Figure 1: 1H-NMR spectrum of the water-soluble melanin booster used in the present invention;

- Figure 2: UV spectrum of the water-soluble melanin booster used in the present invention.

- Figure 3: FT-IR spectrum of the water-soluble melanin booster used in the present invention.

DETAILED DESCRIPTION OF THE INVENTION

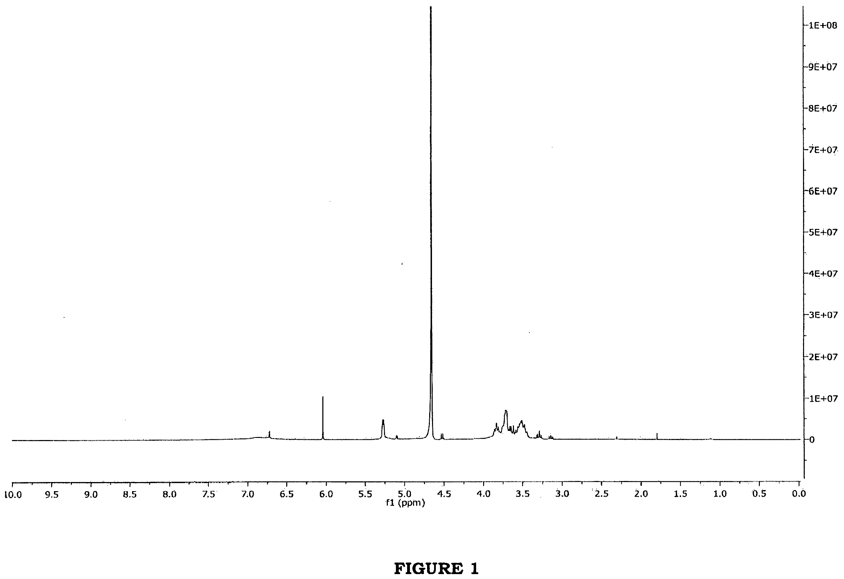

Melanin is the dark pigment present in the skin or a natural or synthetic derivative thereof, absorbing various ranges of the solar UV radiation. Mammalian melanins are highly insoluble in water and, to acquire solubility, require severe treatments such as boiling in strong alkali, or the use of strong oxidants such as hydrogen peroxide, which often damage the melanins. Differently therefrom, the term "water soluble melanin" used herein indicates a melanin having higher solubility in water than in organic solvent (considering $CH_2Cl_2$ as a reference organic solvent), the solubility being measured at 20°C and neutral pH. Typically in the present invention, the water-soluble melanin is obtained by subjecting suitable melanin precursors to enzymatic oxidation by laccase (benzenediol:oxygen oxidoreductase, EC 1.10.3.2): this is a multicopper oxidase able to oxidize a wide variety of substrates with concomitant reduction of oxygen to water (Int.J.Med.Sci., 2020, 21, 966). Preferably, a laccase from *Trametes versicolor* is used; gentisic and/or homogentisic acid are preferred melanin precursors; the enzymatic reaction can be performed in a buffer (e.g. phosphate buffer at pH 7-7.5) with a laccase precursors molar ratio preferably comprised between 1:500 to 1: 1500, e.g. 1:1000; the reaction is conducted for 35-60 hrs (e.g. 48 hrs) under agitation at room temperature and in presence of oxygen; the precursors form radical species which polymerize forming water-soluble melanin; the product is characterizable via UV-VIS, FT-IR, DLS, NMR, CW-pulse and EPR patterns, as e.g. disclosed in Int.J.Mol.Sci., 2021, 22,1739 or in the present Figures 1, 2 and 3.

The composition into which the water-soluble melanin is included is not specifically limited and can be in any physical form suitable for application to a surface of the human body, e.g. a W/O emulsion, O/W emulsion, gel, suspension, cream, spray, powder, foam, etc. In these compositions, the water-soluble melanin can be present at a concentration ranging from 0.001 to 10%, preferably 0.005 to 0.5 wt%, more preferably from 0.003 to 0.3 wt%.

The terms "sunscreen" or "solar filter", or "sun filter", used herein interchangeably, refer to substances that, if applied topically to the skin, protect the same against solar radiation, i.e. exert a photoprotective effect; the effect may comprise absorbing and/or reflecting at least part of sun's ultraviolet (UV) radiation, thus protecting the skin against sun-related damages and diseases, such as erythema, sunburn, skin cancer, etc. Sunscreens can be organic and inorganic. Organic sunscreens absorb UVA and UVB radiation, while inorganic sunscreens reflect UVA (cf. zinc oxide) and UVB (cf. titanium dioxide) radiation. Sunscreens are regulated and only those allowed by the laws of the various countries of the world can be used. The terms sunscreen or solar filter or sun filter used herein do not include melanin. Sunscreens for cosmetic use are widely known and used by the cosmetic formulator, as reviewed e.g. in Am J Clin Dermatol. 2021; 22(6): 819-828. Non-limitative examples thereof are: cinnamates (e.g. DEA Methoxycinnamate, Diisopropyl Methyl Cinnamate, Ethyl Diisopropylcinnamate, Ethylhexyl Methoxycinnamate, Glyceryl Ethylhexanoate Dimethoxycinnamate, Isoamyl-p-methoxycinnamate, Isopentyl Trimethoxycinnamate Trisiloxane, Isopropyl Methoxycinnamate, Cinoxate), triazines (e.g. Bis-Ethylhexyloxyphenol Methoxyphenyl Triazine, Tris-Biphenyl Triazine), triazones (e.g. Ethyhexyl Triazone, Diethylhexyl Butamido Triazone), benzimidazoles (e.g. Disodium Phenyl Dibenzimidazole Tetrasulfonate, Phenylbenzimidazole Sulphonic Acid), camphor derivatives (e.g. 3-Benzylidene Camphor, 4- Methylbenzylidene Camphor, Benzylidene Camphor Sulfonic Acid, Camphor Benzalkonium Methosulphate, Polyacrylamidomethyl Benzylidene Camphor, Terephthalylidene Dicamphor Sulfonic Acid), benzoxazoles, diarylbutadienes, benzotriazoles (e.g. Bisoctrizole, Drometrizole, Drometrizole Trisiloxane), benzylidene malonates, benzal malonates, salicylates (e.g. Ethyl Hexyl Salicylate, Isopropylbenzyl Salicylate, Throlamine Salicylate, Homosalate), benzoates (e.g. Diethylamino Hydroxy Benzoyl Hexyl Benzoate, PABA, Ethyl PABA, Butyl PABA, Ethyl Dihydroxypropyl PABA, Ethylhexyl Dimethyl PABA, Glyceryl PABA, Amyl-p-dimethylaminobenzoate, PEG-25 PABA), benzophenones (e.g. Benzophenone-1, Benzophenone-2, Benzophenone-3, Benzophenone-4, Benzophenone-5, Benzophenone-6, Benzophenone-7, Benzophenone-8, Benzophenone-9, 4-propoxy-2-hydroxybenzophenone), dibenzoylmethanes (e.g. Avobenzone), diphenylacrylates (e.g. Octocrylene), antranylates (e.g. Menthylantranylate), Ferulic Acid, Digalloyl Trioleate, metallic oxides, mixtures of metallic oxydes with lipides (e.g. Titanium Dioxide (and) Caprylic/Capric Triglyceride (and) Alumina (and) Isostearic Acid (and) Polyhydroxystearic Acid (and) Stearic Acid (and) Polyglyceryl-3 Polyricinoleate (and) Lecithin); Zinc Oxide (and) Sesamum Indicum (Sesame) Seed Oil (and) Polyglyceryl-2 Dipolyhydroxystearate (and) Polyglyceryl-3 Diisostearate).

Preferred sunscreens for use in the invention are selected from Butyl Methoxydibenzoylmethane (BMDBM), Octocrylene (OCR), Ethylhexyl Methoxycinnamate (EHMC), Ethylhexyl Salicylate (EHS), Phenylbenzimidazole Sulphonic acid (PBSA), Diethylamino Hydroxybenzoyl Hexylbenzoate (DHHB), Bis-Ethylhexyloxyphenol Methoxyphenyl Triazine (BEMT) or mixtures thereof.

Typical of the present invention is the use of at least two sunscreens, i.e. a combination of sunscreens (also referred herein as "combined sunscreens"), preferably two or three: in fact, in presence of water-soluble melanin and at constancy of total sunscreen amount, these combinations were found more effective as SPF boosters than single sunscreens. Non-limitative examples of combinations of sunscreens according to the invention are: BMDBM/EHMC, BMDBM/EHS, BMDBM/PBSA, BMDBM/OCR, DHHB/OCR, EHMC/BMDBM/OCR, EHMC/DHHB, BMDBM/DHHB/BEMT/EHS, EHMC/DHHB.

Particularly marked enhancements of SPF can be obtained when the combined sunscreens comprise at least one of Butyl Methoxydibenzoylmethane (BMDBM) and/or Octocrylene (OCR); preferred combinations according to this embodiment are: BMDBM/EHMC, BMDBM/EHS, BMDBM/PBSA, BMDBM/OCR, OCR/DHHB, BMDBM/OCR/EHMC, BMDBM/DHHB/BEMT/EHS.

In a specific embodiment, the cosmetic composition is in the form of a O/W emulsion and comprises one of the following combinations of sunscreens: BMDBM/EHMC (e.g. Example 1D), BMDBM/EHS (e.g. Example 1H), BMDBM/PBSA (e.g. Example 1I), BMDBM/OCR (e.g. Example 1N), OCR/DHHB (e.g. Example 1M).

In another specific embodiment, the cosmetic composition is in the form of a W/O emulsion and comprises the sunscreen combination BMDBM/DHHB/ BEMT/ EHS (e,g. Example 2A).

In a further specific embodiment, the cosmetic composition is in the form of a hydrogel and comprises the sunscreen combination BMDBM/EHMC/OCR (e.g. Example 3A).

In all the present embodiments, the combined sunscreens are present at the overall concentration ranging from 0.5 to 80 wt%, e.g. from 0.5 to 30 wt %, or e.g. from 0.5 to 10 wt%.

The present combinations of water-soluble melanin and sunscreens can be formulated in any cosmetic form suitable for application to a surface of the human body, in particular skin or hair. In such compositions, the effect of sun protection can be the primary objective (such as for sunscreen products or self-tanning products) or a secondary objective while other cosmetic effects are primarily sought: non-limitative examples of these forms are facial masks, foundations, face powders, talcum powders, soaps, eau de Cologne, preparations for bath/shower, products for depilation, deodorants, antiperspirants, hair dyes, waving products, straightening and fixing products, styling products, hair cleaning products (lotions, powders, shampoos), hair-shaping or styling products (lotions, lacquers, pomades), shaving products, make-up and de-make-up products, products for application to the lips, nail care products, nail lacquers, skin lightening products and anti-wrinkle products.

A further object of the invention is the use of a water-soluble melanin obtained by enzymatic oxidation of a melanin precursor, as a sun protection factor (SPF) booster for a cosmetic composition comprising sunscreens. Preferred water-soluble melanins, precursors, sunscreeens, respective ratios and concentrations, and types of cosmetic compositions are those detailed above.

A further object of the invention is a process to prepare a cosmetic composition with high sun protection factor (SPF), comprising mixing sunscreens, cosmetic excipients and carriers and a water-soluble melanin obtained by enzymatic oxidation of a melanin precursor. In this further object, the preferred water-soluble melanins, precursors, sunscreens, respective ratios and concentrations are the those detailed above.

The invention is now further disclosed by the following non-limitative examples.

**EXPERIMENTALS**

**Materials and methods**

Various cosmetic formulations were prepared containing physical and chemical sunscreens, such as: W/O emulsions, O/W emulsions, hydrophilic gels. The sunscreens used for the preparation of the formulations are the following:

- *Bis-Ethylhexyloxyphenol Methoxyphenyl Triazine;*

- *Butyl Methoxydibenzoylmethane;*

- *Diethylamino Hydroxybenzoyl Hexyl Benzoate;*

- *Ethylhexyl Methoxycinnamate;*

- *Ethylhexyl Salicylate;*

- *Octocrylene,*

- *Phenylbenzimidazole Sulphonic Acid;*

- *Titanium Dioxide (and) Caprylic/Capric Triglyceride (and) Alumina (and) Isostearic Acid (and) Polyhydroxystearic Acid (and) Stearic Acid (and) Polyglyceryl-3 Polyricinoleate (and) Lecithin;*

- *Water, Ethylhexylmethoxycinnamate, Butyl Methoxydibenzoylmethane, Octocrylene, 1,3 Butylene Glycol, Lecithin, Phenoxyethanol*

- *Zinc Oxide (and) Caprylic/Capric Triglyceride (and) Polyhydroxystearic Acid (and) Isostearic Acid (and) Lecithin (and) Polyglyceryl-3 Polyricinoleate.*

The used SPF booster was water-soluble melanin obtained by laccase oxidation of Gentisic/Homogentisic Acid.

The 1H-NMR spectrum (400 MHz) of the booster is shown in Figure 1.

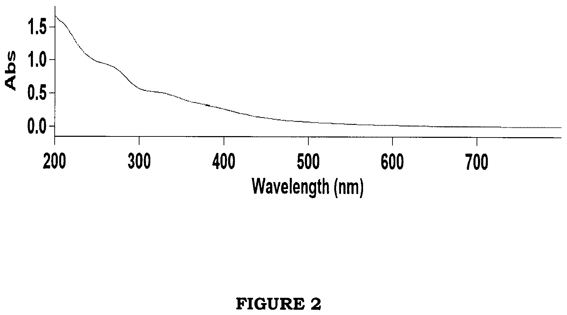

The UV-VIS spectrum of the booster is shown in Figure 2.

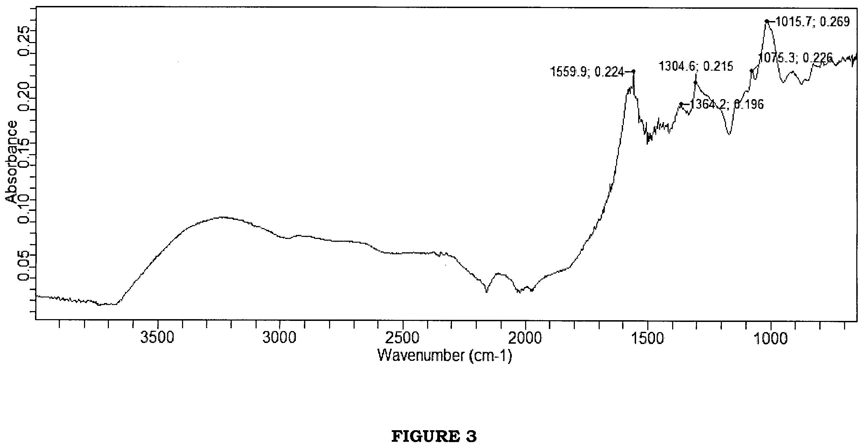

The FT-IR spectrum of the booster is shown in Figure 3.

The SPF value was measured in vitro using the Labsphere 2000S spectrophotometer, capable of providing data based on the diffuse transmittance of UV radiation through the solar product.

The calculation of the SPF value is in accordance with Diffey's equation (Diffley B., Stokes R.P., Serge F., Mazilier C., Rougier A. Suncare product photostability: a key parameter for a more realistic in vitro efficacy evaluation. Eur. J. Dermatol., 7, 226-228 (1987):

$$SPF = \frac{\sum\limits_{290}^{400} E(\lambda) B(\lambda)}{\sum\limits_{290}^{400} E(\lambda) B(\lambda) T(\lambda)}$$

where:

E ($\lambda$) is the spectral solar irradiance;

B ($\lambda$) is the spectrum of erythematous action;

T($\lambda$) is the transmittance of the sample.

For the determination PMMA (Poly Methyl Methacrylate) were used as substrates in the case of W/O emulsions; Transpore ™ and PMMA in the case of O/W emulsions and hydrophilic gels.

## COSMETIC FORMULATIONS

Below are the cosmetic formulations in which the various sunscreens and melanin have been inserted.

### Example 1: O/W emulsions

| | INCI NAME | % |
|---|---|---|
| | *Aqua* | q.b. a 100 |
| | *Sodium Phytate (and) Aqua (and) Alcohol* | 0.1 |
| | *Glycerin* | 4.0 |
| | *Polyglyceryl-3 Rice Branate* | 5.0 |
| | *Dicaprylyl Ether* | 5.0 |
| | *Caprylic/ Capric Triglyceride* | 5.0 |
| | *Glyceryl Stearate* | 3.0 |
| | *Olive Oil Decyl Esters (and) Squalene* | 3.0 |
| | *Olea Europaea Fruit Oil (and) Hydrogenated Vegetable Oil* | 1.0 |
| | *Tocopherol* | 0.1 |
| | *Helianthus Annuus (Sunflower) Seed Oil* | 2.0 |
| | *Behenyl Alcohol* | 2.0 |
| | *Benzyl Alcohol (and) Ethylhexylglycerin (and) Tocopherol* | 1.0 |
| | *Sunscreen* | --- |
| | *Melanin* | --- |

The TABLE 1 below shows the SPF values obtained in the various O\W emulsions (examples 1A-1S) containing the various concentrations of melanin (0.01%, 0.1% e 0.2%) and the relative percent increase of SPF (in brackets):
- **Example 1A:** SUN'CAT MTA 3% (Inci name: *Water, Ethylhexyl Methoxycinnamate, Butyl Methoxydibenzoylmethane, Octocrylene, 1,3 Butylene Glycol, Lecithin, Phenoxyethanol);*
- **Example 1B:** *Ethylhexyl Methoxycinnamate* 6%;
- **Example 1C:** *Butyl Methoxydibenzoylmethane* 3%;
- **Example 1D:** *Ethylhexyl Methoxycinnamate* 6% + *Butyl Methoxydibenzoylmethane* 3%;
- **Example 1E:** *Diethylamino Hydroxybenzoyl Hexylbenzoate* 3%;
- **Example 1F:** *Ethylhexyl Methoxycinnamate* 6% + *Diethylamino Hydroxybenzoyl Hexylbenzoate* 1%;
- **Example 1G:** *Ethylhexyl Methoxycinnamate* 6% + *Diethylamino Hydroxybenzoyl Hexylbenzoate* 3%;
- **Example 1H:** *Ethylhexyl Salicylate* 4% + *Butyl Methoxydibenzoylmethane* 3%;
- **Example 1I:** *Phenylbenzimidazole Sulphonic Acid* 3% + *Butyl Methoxydibenzoylmethane* 3%;
- **Example 1L:** *Octocrylene* 6%;
- **Example 1M:** *Octocrylene* 6% + *Diethylamino Hydroxybenzoyl Hexylbenzoate* 3%;
- **Example 1N:** *Octocrylene* 6% + *Butyl Methoxydibenzoylmethane* 3%;
- **Example IO:** *Bis-Ethylhexyloxyphenol Methoxyphenyl Triazine* 3%;
- **Example 1P:** G-Block DT 200 CCT (Inci name: *Titanium Dioxide (and) Caprylic/Capric Triglyceride (and) Alumina (and) Stearic Acid (and) Isostearic Acid (and) Polyhydroxystearic Acid (and) Polyglyceryl-3 Polyricinoleate (and) Lecithin)* 5% + *Diethylamino Hydroxybenzoyl Hexylbenzoate* 1%;
- **Example 1Q:** G-Block *DT 200 CCT 5%* (Inci name: *Titanium Dioxide (and) Caprylic/Capric Triglyceride (and) Alumina (and) Stearic Acid (and) Isostearic Acid (and) Polyhydroxystearic Acid (and) Polyglyceryl-3 Polyricinoleate (and) Lecithin) + Ethylhexyl Methoxycinnamate* 6% + *Diethylamino Hydroxybenzoyl Hexylbenzoate* 1%.
- **Example 1R:** G- Block DZ 480 CCT 5% (Inci name: Zinc *Oxide (and) Caprylic/Capric Triglyceride (and) Polyhydroxystearic Acid (and) Isostearic Acid (and) Lecithin (and) Polyglyceryl-3 Polyricinoleate)*
- **Example 1S:** G- Block DT 200 CCT 5% + G-Block DZ 480 CCt 5%.

TABLE 1

| Example | SPF | SPF melanin 0.01% | SPF melanin 0.1% | SPF melanin 0.2% |
|---|---|---|---|---|
| 1A | 1.92±0.07 | 2.68±0.19 (39.58%) | 3.22±0.13 (67.71%) | --- |
| 1B | 9.48±0.27 | 11.10±0.21 | 11.32±0.60 | 11.42±0.30 |

(continued)

| Example | SPF | SPF melanin 0.01% (17.09%) | SPF melanin 0.1% (19.41%) | SPF melanin 0.2% (20.46%) |
|---|---|---|---|---|
| 1C | 4.45±0.46 | 4.41±0.26 (---) | 4.47±0.22 (0.45%) | --- |
| 1D | 14.23±0.29 | 23.90±0.74 (67.96%) | 35.52±0.65 (150%) | 59.31±0.33 (317%) |
| 1E | 2.79±0.15 | 2.76±0.27 (---) | 2.51±0.13 (---) | --- |
| 1F | 13.15±0.55 | 14.87±0.48 (13.08%) | 15.74±0.32 (19.69%) | 16,55±0.98 (25.85%) |
| 1G | 15.88±0.35 | 16.41±1.08 (3.34%) | 20.80±0.89 (30.98%) | 22.68±0.65 (42.82%) |
| 1H | 5.80±0.65 | 11.30±0.54 (94.82%) | 15.37±0.65 (165%) | 16.62±0.39 (186.55%) |
| 1I | 11.42±0.26 | 16.07±0.65 (40.72%) | 21.70±0.60 (90%) | 27.87±0.54 (144%) |
| 1L | 7.88±0.08 | --- | 8.10±0.30 (2.79%) | 9.90±0.36 (25.63%) |
| 1M | 10.73±0.29 | 13.18±0.73 (22.83%) | 14.66±0.26 (36.62%) | 25.60±0.19 (138.58%) |
| 1N | 11.50±0.57 | 15.60±0.42 (35.65%) | 18.90±0.53 (64.35%) | 28.00±0.71 (143.48%) |
| 1O | 7.51±0.27 | --- | 10.43±0.77 (38.88%) | 10.58±0.22 (40.88%) |
| 1P | 7.98±0.27 | --- | 9.50±0.35 (19.05%) | 10.53±0.17 (31.95%) |
| 1Q | 20.47±0.44 | --- | 24.31±0.51 (18.76%) | 27.38±0.57 (33.76%) |
| 1R | 2.63±0.30 | ---- | 2.77±0.15 (5.32%) | 2.87±0.22 (9.12%) |
| 1S | 9.22±0.40 | --- | 10.39±0.65 (12.69%) | 10.94±0.93 (18.65%) |

The effect is irrelevant in only a few cases, i.e. with the sunscreen alone (example 1C and 1E). The remaining data reported in the tables show how the combination of sunscreens influences the booster efficacy of melanin. The highest SPF values were obtained with 0.2% melanin with the chemical sunscreens of examples 1D, 1H, 1I, 1M, 1N, where the increase in the sun protection factor varies from 130% up to 320%. In the case of physical sunscreens of Examples 1R and 1S, the increase is around 10-20% while in the case of combining the physical sunscreens and chemical sunscreens in Examples 1P and 1Q the increase is around 20-35% depending on the concentration of melanin.

**Example 2: W/O Emulsions**

| INCI NAME | |
|---|---|
| *Polyglyceryl-6 Pentaoleate* | 3.0 % |
| *Caprylic/Capric Triglyceride* | 4.0 % |
| *C12-15 Alkyl Benzoate* | 7.0 % |
| *Copernicia Cerifera Cera* | 1.5 % |
| *Helianthus Annus (Sunflower) Seed Oil* | 2,0 % |
| *Tocopherol, Lecithin, Ascorbyl palmitate, Citric acid* | 0.05 % |
| *Hydrogenated Polyisobutene* | 6.5 % |
| *Glyceryl Behenate* | 1.0 % |
| *Magnesium Sulfate* | 0.5 % |
| *Glycerin* | 2.5 % |
| *Aqua* | q. b. a 100 |
| *Benzyl Alcohol, Ethylhexylglycerin, Tocopherol* | 1.0 % |

(continued)

| INCI NAME | |
|---|---|
| *Sunscreen* | -- |
| *Melanin* | -- |

The following TABLE 2 shows the SPF values obtained in the W/O emulsions containing the various concentrations of melanin and the relative percentage increases of SPF:

**Example 2A**: *Butyl Methoxydibenzoylmethane* 2.5%. *Diethylaminohydroxybenzoylhexylbenzoate* 2.5%, *Bis-Ethylhexyloxyphenol Methoxyphenyl Triazine* 5.0%, *Ethyl Hexyl Salicylate* 2.0%.

TABLE 2

| Example | SPF | SPF melanin 0.01% | SPF melanin 0.1% | SPF melanin 0.2% |
|---|---|---|---|---|
| 2A | 25.60±0.15 | 30.96±0.29 (20.93%) | 35.60±0.45 (39.06%) | 41.70±0.46 (62.89%) |

From the data reported in the table it can be seen how the mixture of sunscreens influences the booster efficacy of melanin, with SPF increase percentages ranging from 21% to 63% depending on the booster concentration.

**Example 3: hydrophilic gel**

| *INCI NAME* | |
|---|---|
| *Cellulose Gum. Carrageenan, Ceratonia Siliqua Gum, Sucrose* | 1.0 % |
| *Olive Oil Polyglyceryl-4 Esters* | 2.0% |
| *Sodium Phytate (and) Aqua (and) Alcohol* | 0.1 % |
| *Glycerin* | 4.0 % |
| *Aqua* | q. b. a 100 |
| *Benzyl Alcohol, Ethylhexylglycerin, Tocopherol* | 1.0 % |
| *Sunscreen* | -- |
| *Melanin* | -- |

The TABLE 3 shows the SPF values obtained in the formulation of the hydrophilic gel containing the various concentrations of melanin and the relative percentage increases in SPF:

**Example 3A**: SUN'CAT MTA 3% (Inci name: *Water, Ethylhexyl Methoxycinnamate, Butyl Methoxydibenzoylmethane, Octocrylene, 1,3 Butylene Glycol, Lecithin, Phenoxyethanol).*

TABLE 3

| Example | SPF | SPF melanin 0.01% | SPF melanin 0.1% | SPF melanin 0.2% |
|---|---|---|---|---|
| 3A | 3.36±0.18 | 3.64±0.32 (8.33%) | 3.98±0.21 (18.45%) | 5.26±0.46 (56.55%) |

From the data reported in TABLE 3 it can be seen how the mixture of sunscreens influences the booster efficacy of melanin, with SPF increase percentages ranging from 8% up to 57% depending on the booster concentration.

**Example 4 Comparative tests using different melanins**

Different melanins (obtained from laccase oxidation according to the present invention or from a standard commercial source, i.e. melanin from yeast) were added to base sunscreen products, and the % of SPF enhancement after the melanin addition was calculated.

**1: NIVEA SUN SPF 15**

MELANIN FROM LACCASE OXIDATION (INVENTION)

| %MELANIN | SPF | SPF % INCREASE |
|---|---|---|
| 0% | 15.56±0.73 | |
| 0.005% | 19.36 ±1.00 | 24.42% |
| 0.01% | 24.53 ±0.88 | 57.65% |

MELANIN FROM YEAST (REFERENCE)

| % MELANIN | SPF | SPF % INCREASE |
|---|---|---|
| 0% | 17 ±0.19 | |
| 0.0025% | 18 ±0.11 | 5.88% |
| 0.005% | 19 ±0.14 | 11.76% |
| 0.01% | 20 ±0.98 | 17.65% |

**2: GARNIER AMBRE SOLAIRE SPF 15**

MELANIN FROM LACCASE OXIDATION (INVENTION)

| %MELANIN | SPF | SPF % INCREASE |
|---|---|---|
| 0% | 15.66±0.13 | |
| 0.005% | 17.95 ±0.73 | 14.62% |
| 0.01% | 24.68 ±0.67 | 57.60% |

MELANIN FROM YEAST (REFERENCE)

| % MELANIN | SPF | SPF % INCREASE |
|---|---|---|
| 0% | 13 ±0.56 | |
| 0.0025% | 13 ±0.25 | 0% |
| 0.005% | 15 ±0.17 | 15.38% |
| 0.01% | 18 ±1.1 | 38.46% |

**3. NIVEA SUN SPF 20**

MELANIN FROM LACCASE OXIDATION (INVENTION)

| %MELANIN | SPF | SPF % INCREASE |
|---|---|---|
| 0% | 20.98±0.71 | |
| 0.005% | 23.57 ±0.72 | 12.34% |
| 0.01% | 27.24 ±0.64 | 29.83% |

MELANIN FROM YEAST (REFERENCE)

| % MELANIN | SPF | SPF % INCREASE |
|---|---|---|
| 0% | 18 ±0.04 | |
| 0.0025% | 18 ±0.04 | 0% |
| 0.005% | 19 ±0.37 | 5.55% |
| 0.01% | 21 ±0.52 | 16.67% |

**Claims**

1. Cosmetic composition comprising combined sunscreens and a water-soluble melanin obtained by enzymatic oxidation of a melanin precursor.

2. Cosmetic composition according to claim 1 where the enzymatic oxidation is performed by laccase and the melanin precursor is selected from gentisic acid, homogentisic acid and mixtures thereof.

3. Cosmetic composition according to claims 1-2, where the combined sunscreens are organic sunscreens.

4. Cosmetic composition according to claim 3, wherein the combined sunscreens are selected from Butyl Methoxydibenzoylmethane (BMDBM), Octocrylene (OCR), Ethylhexyl Methoxycinnamate (EHMC), Ethylhexyl Salicylate (EHS), Phenylbenzimidazole Sulphonic acid (PBSA), Diethylamino Hydroxybenzoyl Hexylbenzoate (DHHB), Bis-Ethylhexyloxyphenol Methoxyphenyl Triazine (BEMT) or mixtures thereof.

5. Cosmetic composition according to claims 1-4, where the combined sunscreens comprise at least one of Butyl Methoxydibenzoylmethane (BMDBM) and/or Octocrylene (OCR).

6. Cosmetic composition according to claims 1-5, where the combined sunscreens are present at an overall concentration from 0.5 to 80 wt%, e.g. from 0.5 to 30 wt %, or e.g. from 0.5 to 10 wt%.

7. Cosmetic composition according to claims 1-6, where the water-soluble melanin is present at a concentration from 0.001 to 10 wt% .

8. Cosmetic composition according to claims 1-7, where the water-soluble melanin is present at a concentration ranging from 0.005 to 0.5 wt%, preferably from 0.003 to 0.3 wt%.

9. Cosmetic composition according to claims 1-8, in the form of a W/O emulsion, O/W emulsion, hydrogel, gel, suspension, cream, spray, powder or foam.

10. Cosmetic composition according to claim 9, in the form of a O/W emulsion and comprising one of the following sunscreen combinations: BMDBM/EHMC, BMDBM/EHS, BMDBM/PBSA, BMDBM/OCR, OCR/DHHB.

11. Cosmetic composition according to claim 9, in the form of a W/O emulsion and comprising the sunscreen combination BMDBM/DHHB/BEMT/EHS.

12. Cosmetic composition according to claim 9, in the form of a hydrogel and comprising the sunscreen combination BMDBM/EHMC/OCR.

13. Cosmetic composition according to claims 1-12, being formulated as a facial mask, foundation, face powder, talcum powder, soap, eau de Cologne, preparation for bath/shower, product for depilation, deodorant, antiperspirant, hair dye, waving product, straightening and fixing product, styling product, hair cleaning product (lotion, powder, shampoo), hair-shaping or styling product (lotions, lacquers, pomades), shaving product, make-up and de-make-up product, product for application to the lips, nail care products, nail lacquers, sunscreen products, self-tanning products, skin lightening products and anti-wrinkle products.

14. Use of a water-soluble melanin obtained by enzymatic oxidation of a melanin precursor, as a sun protection factor (SPF) booster for a cosmetic composition comprising sunscreens.

15. Process to prepare a cosmetic composition with high sun protection factor (SPF), comprising mixing sunscreens, cosmetic excipients and carriers and a water-soluble melanin obtained by enzymatic oxidation of a melanin precursor.

**FIGURE 1**

FIGURE 2

**FIGURE 3**

Europäisches
Patentamt

European
Patent Office

Office européen
des brevets

# EUROPEAN SEARCH REPORT

Application Number

EP 23 42 5009

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/07696 A1 (ZYLEPSIS LTD [GB]; CHEETHAM PETER SAMUEL JAMES [GB] ET AL.) 31 January 2002 (2002-01-31) * example B(i) * | 1,3,4, 6-10, 13-15 | INV. A61K8/35 A61K8/365 A61K8/368 A61K8/37 |
| X | WO 2021/225657 A2 (AVISA MYKO INC [US]; RAGHUKUMAR SESHAGIRI [IN]) 11 November 2021 (2021-11-11) * paragraphs [0067], [0070]; claim 1 * | 1-15 | A61K8/40 A61K8/46 A61K8/49 A61Q17/04 |
| A | AL KHATIB MAHER ET AL: "Homogentisic Acid and Gentisic Acid Biosynthesized Pyomelanin Mimics: Structural Characterization and Antioxidant Activity", INTERNATIONAL JOURNAL OF MOLECULAR SCIENCES, vol. 22, no. 4, 9 February 2021 (2021-02-09), page 1739, XP093079318, Basel, CH ISSN: 1661-6596, DOI: 10.3390/ijms22041739 * the whole document * | 1-15 | |
| A | US 2022/332670 A1 (ZHOU XUHAO [US] ET AL) 20 October 2022 (2022-10-20) * paragraph [0123] * * paragraph [0189] – paragraph [0214] * | 1-15 | TECHNICAL FIELDS SEARCHED (IPC) A61K A61Q |
| A | US 2004/105826 A1 (SOANE DAVID S [US] ET AL) 3 June 2004 (2004-06-03) * paragraphs [0021], [0024] * | 1-15 | |

The present search report has been drawn up for all claims

| Place of search | Date of completion of the search | Examiner |
|---|---|---|
| Munich | 11 September 2023 | Werner, Stefan |

EPO FORM 1503 03.82 (P04C01)

## ANNEX TO THE EUROPEAN SEARCH REPORT
## ON EUROPEAN PATENT APPLICATION NO.

EP 23 42 5009

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2023

| Patent document cited in search report | | Publication date | Patent family member(s) | | Publication date |
|---|---|---|---|---|---|
| WO 0207696 | A1 | 31-01-2002 | AT | 353008 T | 15-02-2007 |
| | | | AU | 6930101 A | 05-02-2002 |
| | | | DE | 60126412 T2 | 25-10-2007 |
| | | | EP | 1301165 A1 | 16-04-2003 |
| | | | ES | 2281425 T3 | 01-10-2007 |
| | | | WO | 0207696 A1 | 31-01-2002 |
| WO 2021225657 | A2 | 11-11-2021 | CA | 3164397 A1 | 11-11-2021 |
| | | | US | 2023106900 A1 | 06-04-2023 |
| | | | WO | 2021225657 A2 | 11-11-2021 |
| US 2022332670 | A1 | 20-10-2022 | EP | 3990423 A2 | 04-05-2022 |
| | | | US | 2022332670 A1 | 20-10-2022 |
| | | | WO | 2021021350 A2 | 04-02-2021 |
| US 2004105826 | A1 | 03-06-2004 | BR | 0210979 A | 08-06-2004 |
| | | | CA | 2448252 A1 | 19-12-2002 |
| | | | CN | 1525847 A | 01-09-2004 |
| | | | EP | 1399123 A2 | 24-03-2004 |
| | | | JP | 2004537528 A | 16-12-2004 |
| | | | KR | 20040025925 A | 26-03-2004 |
| | | | MX | PA03011311 A | 06-12-2004 |
| | | | US | 2004105826 A1 | 03-06-2004 |
| | | | WO | 02100371 A2 | 19-12-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080219938 A1
- EP 2512434 B1
- US 20110256076 A1
- AU 2009307763 B2
- WO 2012161084 A1
- WO 2015014818 A2
- WO 201607136 A1
- WO 2019024022 A1
- WO 2020024023 A1
- WO 2020041391 A1
- KR 101995997
- JP 5837868 B

**Non-patent literature cited in the description**

- *Int.J.Med.Sci.,* 2020, vol. 21, 966
- *Int.J.Mol.Sci.,* 2021, vol. 22, 1739
- *Am J Clin Dermatol.,* 2021, vol. 22 (6), 819-828
- **DIFFLEY B. ; STOKES R.P. ; SERGE F. ; MAZILIER C. ; ROUGIER A.** Suncare product photostability: a key parameter for a more realistic in vitro efficacy evaluation. *Eur. J. Dermatol.,* 1987, vol. 7, 226-228